# EUROPÄISCHE PATENTSCHRIFT

(11) **EP 0 537 687 B1**
(45) Veröffentlichungstag und Bekanntmachung des Hinweises auf die Patenterteilung: **12.03.1997**
(21) Anmeldenummer: 92117478.5
(22) Anmeldetag: 13.10.1992
(51) Int. Cl.: D06M 13/137

(54) **Biologisch abbaubare Faserpräparationsmittel**
Biodegradable fiber treating agent
Agent de traitement de fibres biodégradables

(30) Priorität: 15.10.1991 DE 4134113
(43) Veröffentlichungstag der Anmeldung: 21.04.1993
(73) Patentinhaber: HOECHST AKTIENGESELLSCHAFT, 65926 Frankfurt am Main (DE)
(72) Erfinder: Kleber, Rolf, Dr., W-6078 Neu-Isenburg (DE); Weinelt, Frank, Dr., W-8269 Burgkirchen (DE); Jaeckel, Lothar, W-6093 Flörsheim/Main (DE); Oberhauser, Adelgunde, W-8265 Neuötting (DE)

(56) Entgegenhaltungen:
- DE-C- 3 936 975
- GB-A- 2 017 100
- US-A- 2 068 003
- US-A- 2 249 519

## Beschreibung

Faserpräparationsmittel werden auf Textilfasern, Filamenten oder Stapelfasern beim Herstellen und Weiterverarbeiten aufgebracht, um die Neigung zu Faser- oder Filamentbrüchen bei der mechanischen Beanspruchung zu mindern. Die Faserpräparationsmittel erleichtern die Abläufe beim Herstellen, wie dem Aufwickeln, Verstrecken, Kräuseln, Texturieren, Weben, Kardieren, und Stricken.

Acetale als Faserpräparationsmittel für Celluloseregeneraffasern sind bereits aus US-A-2 068 003 bekannt. Diese Acetale basieren auf Polyalkoholen.

In den letzten Jahren werden an Faserpräparationsmittel zusätzliche Bedingungen dahingehend gestellt, daß diese Mittel im Abwasser biologisch gut abbaubar sein sollen. Diese Bedingungen zielen darauf ab, die in die Abwässer von Textilbetrieben beim Färben oder Vorbehandeln gelangenden Faserpräparationsmittel durch biologischen Abbau zu eliminieren. Unter dem Begriff "biologisch abbaubar" ist zu verstehen, daß die Bestandteile von Faserpräparationsmitteln, wie Gleitmittel, Tenside, Fadenschlußmittel oder auch Antistatika, möglichst vollständig oder zumindest zu 70 Gew.-% auf biologischem Weg, z.B. durch die in dem Klärschlamm einer Kläranlage enthaltenen Enzyme oder Bakterien, abgebaut werden. Dabei ist es wünschenswert, daß bei dem Abbau chemisch einfache Verbindungen, wie Kohlendioxid, Wasser, Sulfat oder Phosphat, entstehen.
Das Ausmaß der biologischen Abbaubarkeit kann beispielsweise anhand der gebildeten Metabolite oder der biologischen Elimination bestimmt werden. Die Fachliteratur nennt verschiedene Tests zur Prüfung der biologischen Abbaubarkeit, wie den Flußwasser-Test (OECD 301 C-Test), den Zahn-Wellens-Test (OECD-302-B-Test) oder auch den Sturm-Test.
Die üblicherweise als Faserpräparationsmittel speziell für Filamente eingesetzten Gleitmittel/Tensid-Gemische sind von seiten der Tenside zunehmend biologisch gut abbaubar, jedoch sind die bisherigen nichttensidischen Gleitmittel auf Basis von Mineralölen, wie Weißöle, oder synthetischen Estern, wie Pentaerythrittetrapelaronat biologisch kaum oder gar nicht abbaubar. Zu den biologisch gut abbaubaren Tensiden zählen Fettalkoholoxethylate. In der US-A-3 926 816 werden speziell Spulöle auf Basis biologisch nicht abbaubarer Gleitmittel vom Typ Alkylfettsäureester mit C₁₀-C₁₈-Fettalkohol und C₆-C₂₂-Fettsäure beschrieben.
Andere Gleitmittel, die biologisch ebenfalls nicht abbaubar sind, sind inerte wasserunlösliche Mineralöle niederer Viskosität.

Die Bereitstellung biologisch abbaubarer Faserpräparationsmittel bereitet nach wie vor große Schwierigkeiten. Hier will die Erfindung Abhilfe schaffen.

Faserpräparationsmittel, gekennzeichnet durch einen Gehalt an 10 bis 90 Gew.-Teilen der Verbindungen der Formel 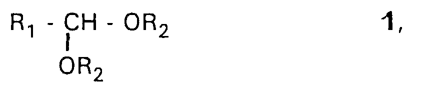 in welcher
- R₁: ein geradkettiger oder verzweigter Alkylrest der Summenformel CₙH₂ₙ₊₁ oder ein geradkettiger oder verzweigter Alkenylrest der Summenformel CₙH₂ₙ₋₁ mit jeweils n gleich 1 bis 9 und
- R₂: ein geradkettiger oder verzweigter Alkylrest der Summenformel CₙH₂ₙ₊₁ mit n gleich 2 bis 16 sind oder 10 bis 90 Gew.-Teile Isobutyraldehyd-dibutylglycolacetal
und
90 bis 10 Gew.-Teilen an biologisch abbaubaren Tensiden und gegebenenfalls anderen Faserpräparationsmitteln, bezogen auf das Gewicht der Faserpräparationsformulierung.

Die Acetale sind aus entsprechenden Alkoholen und Aldehyden leicht durch saure Katalyse herzustellen. Zunächst werden der Alkohol und Aldehyd in einem Lösemittel, das gleichzeitig als Schleppmittel für das bei der Reaktion gebildete Wasser dient, gelöst. Der Siedebereich des Lösungsmittels liegt unterhalb 100 °C, bevorzugt zwischen 30 und 90 °C. Geeignete Lösungsmittel sind aliphatische Kohlenwasserstoffe wie n-Hexan, Kohlenwasserstoffgemische, wie Petrolether oder halogenierte Kohlenwassertoffe, wie Dichlormethan oder Chloroform. Um möglichst hohe Ausbeuten an Acetalen zu erzielen, wird der Alkohol im Überschuß zugegeben. Das Verhältnis von Aldehyd zu Alkohol liegt im

Bereich von 1 : 1,1 - 6, bevorzugt 1 : 4. Als Katalysatoren werden die zur Herstellung von Acetalen üblichen sauren Katalysatoren verwendet, wie Schwefelsäure oder p-Toluolsulfonsäure. Anschließend wird die Lösung solange unter Rückfluß erhitzt bis kein Reaktionswasser mehr abgeschieden wird. Nach Beendung der Umsetzung wird die Lösung mit einer 2 - 5 gew.-%igen Natriumalkoholatlösung, bevorzugt Natriummethylatlösung auf einen pH-Wert im Bereich von etwa 7 - 9 eingestellt. Durch anschließende Destillation werden aus der alkalischen Lösung das Schleppmittel und der unverbrauchte Alkohol entfernt. Das Acetal verbleibt als flüssiger meist öliger Rückstand und wird von dem anfallenden festen Natriumalkoholat abgetrennt z. B. durch Filtration. Falls erforderlich kann das Acetal durch Destillation gereinigt werden.

Diese Acetale besitzen eine Reihe von vorteilhaften Eigenschaften, die eine breite Anwendbarkeit auf dem Gebiet der Faserherstellung und der Weiterverarbeitung von Fasern ermöglichen.
So hat es sich gezeigt, daß diese Acetale trotz ihrer Verzweigung in der Acetal-CHO-Gruppe und ihrer oftmals geringen Wasserlöslichkeit eine gute biologische Abbaubarkeit besitzen. Die Wasserlöslichkeit liegt im Bereich von 10 bis 100 Gew.-%. Der Grad der biologischen Abbaubarkeit der Faserpräparationsmittel kann im Falle der Acetale durch den OECD 302-B-Test bestimmt werden.
Dabei hat sich gezeigt, daß der Grad der biologischen Abbaubarkeit im Bereich von bis zu 100 Gew.-% an eingesetzten Acetalen liegt.

Es hat sich außerdem gezeigt, daß die Acetale ausgeprägte Gleiteigenschaften besitzen und als Spulöle eingesetzt werden können, wobei sie die Reibechtheit von gefärbten Fasern, insbesondere dispersionsgefärbten Polyesterfasern, nicht herabsetzen. Dabei können die Spulöle verschiedene Zusammensetzungen besitzen. So können die Spulöle nur aus den Acetalverbindungen bestehen (neat-oil application) oder unter Zusatz von 1 - 20 Gew.-% eines Tensids zur Verbesserung der Auswaschbarkeit der Spulöle auf die Faser aufgetragen werden. Bei den eingesetzten Tensiden handelt es sich um die üblicherweise für Spulöl verwendeten Tenside, die aber die Eigenschaft der biologischen Abbaubarkeit besitzen sollen.

Neben ihrer Verwendung als Spulöle können die erfindungsgemäßen Acetale sowohl allein, als auch in Mischung untereinander oder mit anderen, an sich bekannten Faserpräparationsmitteln, wie Tensiden, Antistatika vom Typ der P₂O₅-Estersalze, Fadenschlußmittel und anderen, als Faserpräparationsmittel eingesetzt werden.
Bei der Verwendung einer Mischung der Acetale mit bekannten Faserpräparationsmitteln soll der Anteil dieser Verbindungen wenigstens 10 Gew.-Teile, bevorzugt mehr als 30 Gew.-Teile und besonders bevorzugt mehr als 70 Gew.-Teile betragen.
Der Anteil an biologisch abbaubaren Tensiden im Faserpräparationsmittel liegt im Bereich von 90 bis 10 Gew.-Teilen.

Bei der Präparation von Synthesefasern mit den Acetalen oder deren Mischungen mit anderen Faserpräparationsmitteln beträgt die Gesamtauflage von etwa 0,1 bis 4 Gew.-%, vorzugsweise von etwa 1 bis 2 Gew.-%, bezogen auf das Gewicht der Faser. Das Aufbringen auf die Faser erfolgt nach üblichen Methoden, beispielsweise durch Tauchen, Sprühen, Foulardieren oder Pflatschen, beim Spinnen, Verstrecken oder als Finalpräparation mittels Galetten. Die Faserpräparationsmittel können wie üblich aus Wasser als Lösung oder Dispersion, gegebenenfalls unter Verwendung geeigneter Löse- oder Dispergiermittel, wie Petrolether, aufgebracht werden.
Als Fäden und Fasern können Endlosfäden, Stapelfasern, Kabel oder Füllfaser eingesetzt werden.
Als Synthesefasern, für die die Faserpräparationsmittel gemäß der Erfindung anzuwenden sind, kommen z.B. Fasern aus Polyestern, Polyamiden, Polyacrylnitril, Polyolefinen, wie Polyethylen oder Polypropylen, oder den Copolymeren auf Basis der vorstehend genannten Verbindungen in Betracht. Bevorzugt finden die Verbindungen der Formel 1 Anwendung für die Präparation von Polyesterfasern.

Nachfolgend soll die Erfindung anhand von Beispielen erläutert werden.

Allgemeine Vorschrift zur Herstellung der in den nachfolgenden Beispielen genannten Acetale I bis VII:
3,5 Mol Aldehyd, 14 Mol Alkohol, 500 g n-Hexan, Methylenchlorid oder Chloroform werden gemischt, mit 1 g p-Toluolsulfonsäure als Katalysator versetzt und zum Sieden erwärmt. Das entstehende Wasser wird azeotrop abdestilliert. Nach beendeter Wasserbildung stellt man mit Na-methylat-Lösung alkalisch und destilliert anschließend das Schleppmittel und den unverbrauchten Alkohol ab. Das Acetal wird von ausgefallenem Salz filtriert und kann wahlweise destilliert werden.

Die physikalischen Daten der Acetale I bis VII sind in Tabelle A aufgeführt.

**Tabelle A**

| Physikalische Daten der Beispiele I bis VII | | | | |
|---|---|---|---|---|
| | Bezeichnung | Kp. | n_{D}²¹ | Viskosität mPa.s,15°C |
| I | Butyraldehyddi-n-butylacetal | 196°C | 1.4160 | 1.9 |
| II | Acetaldehyddi-2-ethyl-hexylacetal | 269°C | 1.4350 | 5.19 |
| III | Acetaldehyddi-n-hexylacetal | 246°C | 1.4235 | 2.76 |
| IV | Isobutyraldehyddi-n-hexylacetal | 251°C | 1.4270 | 3.8 |
| V | n-Butyraldehyddi-n-octylacetal | 224°C | 1.4370 | 7.6 |
| VI | Isobutyraldehyddi-n-octylacetal | 230°C | 1.4351 | 7.7 |
| VII | Isobutyraldehyddibutylglycolacetal | 272°C | 1.4285 | 4.5 |

Prüfung der biologischen Abbaubarkeit der Faserpräparationsmittel IX bis XVI:

Die Prüfung der biologische Abbaubarkeit der Faserpräparationsmittel IX bis XVI erfolgt nach dem OECD 302-B-Test. Die Faserpräparationsmittel IX bis XVI bestehen jeweils aus 90 Gew.-Teilen eines der Acetale I bis VIII und 10 Gew.-Teilen Cocosfettalkohol (5EO).
(EO bedeutet Ethylenoxideinheiten)

Die Faserpräparationsmittel XVII und XVII werden zum Vergleich mit den erfindungsgemäßen Faserpräparationsmittel IX bis XVI ebenfalls auf ihre biologische Abbaubarkeit geprüft.

### Bezeichnung

- XVII: Mineral-Öl / Cocosfettalkohol (5 EO) 90 Gew.-Teile / 10 Gew.-Teile
- XVIII: Butylstearat /Isotridecylalkohol (7 EO) 95 Gew.-Teile / 5 Gew.-Teile

Tabelle B zeigt das Ausmaß der biologischen Abbaubarkeit.

**Tabelle B**

| Faserpräparationsmittel | Biologische Abbaubarkeit |
|---|---|
| IX - XVI | > 80% |
| XVII | < 30% |
| XVIII | < 25% |

Prüfung der Reibechtheit und der Auswaschbarkeit:
Ein Wirkschlauch aus texturiertem Polyester (dtex 1676 f 32), der mit einem Dispersionsfarbstoff blau gefärbt wird, wird mit den nachfolgend aufgeführten Faserpräparationsmitteln behandelt.

### Bezeichnung

- IX: Butyraldehyddi-n-butylacetal / Cocosfettalkohol (5 EO) 90 Gew-Teile / 10 Gew.-Teile
- XVII: Mineral-Öl / Cocosfettalkohol (5 EO) 90 Gew.-Teile / 10 Gew.-Teile
- XVIII: Butylstearat /Isotridecylalkohol (7 EO) 95 Gew.-Teile / 5 Gew.-Teile

Der Auftrag der Faserpräparationsmittel erfolgt mit einer Auflage von 3 Gew.-%, bezogen auf das Gewicht des Wirkschlauches mittels Galetten.

### Prüfung der Reibechtheit:

Die so präparatierten Wirkschläuche werden unfixiert bzw. nach einer Fixierung bei einer Temperatur von 180°C über einen Zeitraum von 30 Sekunden und nach einer Lagerzeit von 14 Tagen, bei einer Temperatur von 20°C und einer Luftfeuchte von 60%, auf ihre Reibechtheiten nach DIN-54021 mit einem Crockmeter geprüft.
Die Prüfung der Reibechtheit erfolgt an den trockenen und mit elektrolytfreiem Wasser befeuchteten Wirkschläuchen.
Die Beurteilung der Reibechtheit erfolgt mit hierfür vorgesehenen Vergleichstandards, wobei die Vergleiche mit den Zahlen 1 (schlechte Reibechtheit) bis 6 (sehr gut), analog dem umgekehrten Schulnotensystem, bewertet werden.

### Prüfung der Auswaschbarkeit:

Zur Prüfung der Auswaschbarkeit werden die vorstehend genannten unfixierten und fixierten Wirkschläuche über einen Zeitraum von 30 min bei einer Temperatur von 60°C im Flottenverhältnis 1:10 mit 3 g/l einer wäßrigen Natriumlaurylsulfatlösung gewaschen und die Auswaschbarkeit im UV-Licht beurteilt.
Die Beurteilung der Auswaschbarkeit erfolgt analog der Beurteilung der Reibechtheit und ist ebenfalls in Tabelle C angegeben.

**Tabelle C**

| Bezeichnung | Reibechtheiten unfixiert | | Reibechtheiten fixiert | | Auswaschbarkeit |
|---|---|---|---|---|---|
| | trocken | naß | trocken | naß | fixiert |
| IX | 6 | 6 | 5 | 5 | 6 |
| XVII | 4 | 4 | 3 | 3 | 4 |
| XVII | 3 | 2 | 2 | 2 | 3 |

## Patentansprüche

1. Faserpräparationsmittel, gekennzeichnet durch einen Gehalt an 10 bis 90 Gew.-Teilen der Verbindungen der Formel 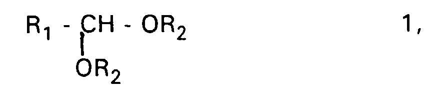 in welcher
R₁ ein geradkettiger oder verzweigter Alkylrest der Summenformel CₙH₂ₙ₊₁ oder ein geradkettiger oder verzweigter Alkenylrest der Summenformel CₙH₂ₙ₋₁ mit jeweils n gleich 1 bis 9 und
R₂ ein geradkettiger oder verzweigter Alkylrest der Summenformel CₙH₂ₙ₊₁ mit n gleich 2 bis 16 sind oder 10 bis 90 Gew.-Teile Isobutyraldehyddibutylglycolacetal
und
90 bis 10 Gew.-Teilen an biologisch abbaubaren Tensiden und gegebenenfalls anderen Faserpräparationsmitteln, bezogen auf das Gewicht der Faserpräparationsformulierung.

2. Mittel nach Anspruch 1, gekennzeichnet durch einen Gehalt an Verbindungen der Formel 1, in welcher R₁ und R₂ gleich und ein geradkettiger oder verzweigter Alkylrest mit n = 1 bis 9 sind.

3. Mittel nach Anspruch 1 oder 2, gekennzeichnet durch einen Gehalt an Verbindungen der Formel 1, in welcher R₁ gleich Methyl und R₂ gleich n-Hexyl oder R₁ gleich Isobutyl und R₂ gleich Isooctyl oder n-Hexadecyl sind.

4. Mittel nach einem der Ansprüche 1 bis 3, dadurch gekennzeichnet, daß die biologische Abbaubarkeit mindestens 70 Gew.-%, bezogen auf das Faserpräparationsmittel, beträgt.

5. Verwendung der Mittel gemäß einem der Ansprüche 1 bis 4 als Spulöle für Synthesefasern.

6. Verwendung nach Anspruch 5, dadurch gekennzeichnet, daß die Synthesefasern aus Polyester, Polyacrylamid, Polyamid, Polyolefinen, wie Polyethylen und Polypropylen, und den Copolymeren auf Basis der vorstehend genannten Verbindungen bestehen.

7. Verwendung von Verbindungen der Formel 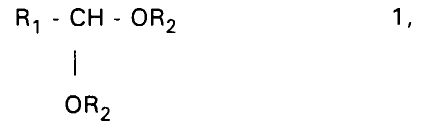 in welcher
R₁ ein geradkettiger oder verzweigter Alkylrest der Summenformel CₙH₂ₙ₊₁ oder ein geradkettiger oder verzweigter Alkenylrest der Summenformel CₙH₂ₙ₋₁ mit jeweils n gleich 1 oder 9 und
R₂ ein geradkettiger oder verzweigter Alkylrest der Summenformel CₙH₂ₙ₊₁ mit n gleich 2 bis 16 sind oder
Isobutyraldehyddibutylglycolacetal,
als Faserpräparationsmittel.

8. Verwendung nach Anspruch 7, dadurch gekennzeichnet, daß in den Verbindungen der Formel 1 R¹ und R² gleich und ein geradkettiger oder verzweigter Alkylrest mit n = 1 bis 9 sind.

9. Verwendung nach Anspruch 7 oder 8, dadurch gekennzeichnet, daß in den Verbindungen der Formel 1 R¹ gleich Methyl und R² gleich n-Hexyl oder R¹ gleich Isobutyl und R² gleich Isooctyl oder n-Hexadecyl sind.

10. Verwendung nach einem der Ansprüche 7 bis 9, dadurch gekennzeichnet, daß 10 bis 90 Gew.-Teile der Verbindungen der Formel 1 und
90 bis 10 Gew.-Teile an biologisch abbaubaren Tensiden und gegebenenfalls anderen Faserpräparationsmitteln, bezogen auf das Gewicht der Faserpräparationsformulierung, eingesetzt werden.

11. Verwendung nach einem der Ansprüche 7 bis 10 dadurch gekennzeichnet, daß die Faserpräparationsmittel Spulöle für Synthesefasern sind.

12. Verwendung nach Anspruch 11, dadurch gekennzeichnet, daß die Synthesefasern aus Polyester, Polyacrylamid, Polyamid, Polyolefinen, wie Polyethylen und Polypropylen, und den Copolymeren auf Basis der vorstehend genannten Verbindungen bestehen.

## Claims

1. A spin finish, comprising 10 to 90 parts by weight of compounds of the formula 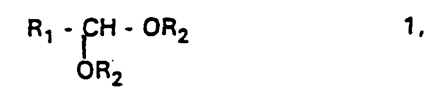 in which
R₁ is a straight-chain or branched alkyl radical of the empirical formula cₙH₂ₙ₊₁ or a straight-chain or branched alkenyl radical of the empirical formula CₙH₂ₙ₋₁ where n is in each case 1 to 9 and
R₂ is a straight-chain or branched alkyl radical of the empirical formula CₙH₂ₙ₊₁ where n is 2 to 16, or 10 to 90 parts by weight of isobutyraldehyde dibutylglycol acetal
and
90 to 10 parts by weight of biodegradable surfactants and, if desired, other spin finishes, relative to the weight of the spin finish formulation.

2. The finish as claimed in claim 1, comprising compounds of the formula 1 in which R₁ and R₂ are identical and a straight-chain or branched alkyl radical where n is 1 to 9.

3. The spin finish as claimed in claim 1 or 2, comprising compounds of the formula 1 in which R₁ is methyl and R₂ is n-hexyl or R₁ is isobutyl and R₂ is isooctyl or n-hexadecyl.

4. The spin finish as claimed in one of claims 1 to 3, wherein the biodegradability is at least 70% by weight, relative to the spin finish.

5. Use of the spin finish as claimed in one of claims 1 to 4 as spooling oil for synthetic fibers.

6. Use as claimed in claim 5, wherein the synthetic fibers are made of polyester, polyacrylamide, polyamide, polyolefins, such as polyethylene and polypropylene, and copolymers based on the abovementioned compounds.

7. Use of compounds of the formula 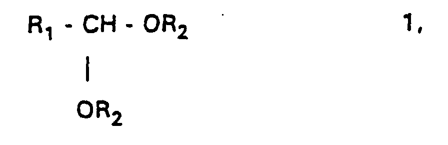 in which
R₁ is a straight-chain or branched alkyl radical of the empirical formula CₙH₂ₙ₊₁ or a straight-chain or branched alkenyl radical of the empirical formula CₙH₂ₙ₋₁ where n is in each case 1 to 9 and
R₂ is a straight-chain or branched alkyl radical of the empirical formula CₙH₂ₙ₊₁ where n is 2 to 16, or of isobutyraldehyde dibutylglycol acetal
as spin finish.

8. Use as claimed in claim 7, wherein in the compounds of the formula 1 R₁ and R₂ are identical and a straight-chain or branched alkyl radical where n is 1 to 9.

9. Use as claimed in claim 7 or 8, wherein in the compounds of the formula 1 R₁ is methyl and R₂ is n-hexyl or R₁ is isobutyl and R₂ is isooctyl or n-hexadecyl.

10. Use as claimed in one of claims 7 to 9, wherein 10 to 90 parts by weight of compounds of the formula 1 and
90 to 10 parts by weight of biodegradable surfactants and, if desired, other spin finishes, relative to the weight of the spin finish formulation, are used.

11. Use as claimed in one of claims 7 to 10, wherein the spin finish is used as spooling oil for synthetic fibers.

12. Use as claimed in claim 11, wherein the synthetic fibers are made of polyester, polyacrylamide, polyamide, polyolefins, such as polyethylene and polypropylene, and copolymers based on the abovementioned compounds.

## Revendications

1. Agents de traitement de fibres, caractérisés par une teneur de 10 à 90 parties en poids des composés de formule 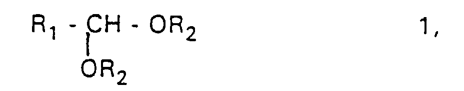 dans laquelle
R₁ est un radical alkyle, rectiligne ou ramifié, de formule brute CₙH₂ₙ₊₁ ou un radical alcényle, rectiligne ou ramifié, de formule brute CₙH₂ₙ₋₁, avec dans chaque cas n représentant 1 à 9 et
R₂ est un radical alkyle, rectiligne ou ramifié, de formule brute CₙH₂ₙ₊₁ avec n représentant 2 à 16
ou 10 à 90 parties en poids d'acétal d'aldéhyde isobutyrique et de dibutylglycol
et
90 à 10 parties en poids d'agents tensio-actifs biodégradables et éventuellement d'autres agents de traitement de fibres, par rapport au poids de la formulation de traitement de fibres.

2. Agents selon la revendication 1, caractérisés par une teneur en composés de formule 1 dans laquelle R₁ et R₂ sont identiques et sont un radical alkyle, rectiligne ou ramifié, avec n = 1 à 9.

3. Agents selon la revendication 1 ou 2, caractérisés par une teneur en composés de formule 1 dans laquelle R₁ représente méthyle et R₂ représente n-hexyle ou R₁ représente isobutyle et R₂ représente isooctyle ou n-hexadécyle.

4. Agents selon l'une des revendications 1 à 3, caractérisés en ce que la biodégradabilité est au moins de 70% en poids, par rapport à l'agent de traitement de fibres.

5. Utilisation des agents selon une des revendications 1 à 4 comme huiles de bobinage pour des fibres synthétiques.

6. Utilisation selon la revendication 5, caractérisée en ce que les fibres synthétiques sont constituées de polyester, polyacrylamide, polyamide, polyoléfines comme le polyéthylène et le polypropylène, ou des copolymères à base des composés susmentionnés.

7. Utilisation des composés de formule 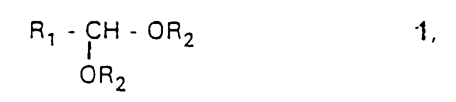 dans laquelle
R₁ est un radical alkyle, rectiligne ou ramifié, de formule brute CₙH₂ₙ₊₁ ou un radical alcényle, rectiligne ou ramifié, de formule brute CₙH₂ₙ₋₁, avec dans chaque cas n représentant 1 à 9 et
R₂ est un radical alkyle, rectiligne ou ramifié, de formule brute CₙH₂ₙ₊₁ avec n représentant 2 à 16 ou
l'acétal dibutylglycolique de l'aldéhyde isobutyrique, comme agents de traitement de fibres.

8. Utilisation selon la revendication 7, caractérisée en ce que dans les composés de formule 1 R₁ et R₂ sont identiques et sont un radical alkyle, rectiligne ou ramifié, avec n = 1 à 9.

9. Utilisation selon la revendication 7 ou 8, caractérisée en ce que dans les composés de formule 1 R₁ représente méthyle et R₂ représente n-hexyle ou R₁ représente isobutyle et R₂ représente isooctyle ou n-hexadécyle.

10. Utilisation selon une des revendications 7 à 9, caractérisée en ce que 10 à 90 parties en poids des composés de formule 1
et
90 à 10 parties en poids d'agents tensio-actifs biodégradables et éventuellement d'autres agents de traitement de fibres, par rapport au poids de la formulation de traitement de fibres, sont mis en oeuvres.

11. Utilisation selon une des revendications 7 à 10, caractérisée en ce que les agents de traitement de fibres sont des huiles de bobinage pour des fibres synthétiques.

12. Utilisation selon la revendication 11, caractérisée en ce que les fibres synthétiques sont constituées de polyester, polyacrylamide, polyamide, polyoléfines comme le polyéthylène ou le polypropylène, ou des copolymères à base des composés susmentionnés.
